Europäisches Patentamt

European Patent Office

Office européen des brevets

(19)

(11) Veröffentlichungsnummer: **0 304 000**
**A1**

(12) **EUROPÄISCHE PATENTANMELDUNG**

(21) Anmeldenummer: 88113215.3

(22) Anmeldetag: 13.08.88

(51) Int. Cl.⁴: **G01N 33/543 , B01L 3/00**

(30) Priorität: 19.08.87 DE 3727590
05.08.88 DE 8810037 U

(43) Veröffentlichungstag der Anmeldung:
22.02.89 Patentblatt 89/08

(84) Benannte Vertragsstaaten:
AT BE CH DE ES FR GB GR IT LI LU NL SE

(71) Anmelder: FLEMMING GMBH
Im Maisel 14
D-6204 Taunusstein 4(DE)

(72) Erfinder: Brodt, Rainer
Am Hirtenhaus 4B
D-6209 Heidenrod-Kemel(DE)
Erfinder: Gorka, Günther, Dr.-Chem.
Adalbert-Stifter-Strasse 10
D-6270 Idstein(DE)
Erfinder: Fehse, Peter
Am Steinberg 85
D-6057 Dietzenbach 2(DE)
Erfinder: Reid, John, Dr.
14 Hillcrest, Roslevan, Ennis
County Clare(IE)
Erfinder: Holmes, Steve, Dr.
Gortaganniv, Kilmaley
County Clare(IE)

(74) Vertreter: Weber, Dieter, Dr. et al
Willrath Weber und Seiffert Postfach 6145
Gustav-Freytag-Strasse 25
D-6200 Wiesbaden 1(DE)

(54) Verfahren und Vorrichtung zur Ermittlung eines Antigens oder Antikörpers in einer flüssigen Probe.

(57) Ein Verfahren zur Ermittlung eines Antigens oder Antikörpers in einer flüssigen Probe, bei dem man die Probe mit einer porösen Membran, an die ein entsprechender Antikörper bzw. entsprechendes Antigen gebunden ist und an die sich ein Flüssigkeit aufnehmendes poröses Absorbensmaterial anschließt, und einem sich an das Antigen bzw. den Antikörper bindenden konjugierten oder gelabelten Antikörper in Berührung bringt und gegebenenfalls diese Antikörper unter Bildung eines Farbstoffes entwickelt, ist dadurch gekennzeichnet, daß man in einem Behälter ein solches Volumen der flüssigen Probe, daß mit ihm das Porenvolumen des Absorbensmaterials nur teilweise füllbar ist, vorliegt, die Membran mit dem daran anschließenden Absorbensmaterial derart in die flüssige Probe eintaucht, daß diese im wesentlichen vollständig oder mit vorbestimmtem Volumen entgegen der Schwerkraft durch die Membran in das Absorbensmaterial gesaugt wird, und gegebenenfalls schließlich nach Entfernung der Membran aus der Probe sie in eine Entwicklerlösung eintaucht und diese entgegen der Schwerkraft in das Absorbensmaterial einsaugen läßt. Die Vorrichtung für das Verfahren besitzt einen Probenbehälter und einen rohrförmigen Absorbensmaterial-Behälter.

Fig. 2

## Verfahren und Vorrichtung zur Ermittlung eines Antigens oder Antikörpers in einer flüssigen Probe

Es gibt eine Reihe bekannter Verfahren zur Ermittlung eines Antigens in einer flüssigen Probe, bei denen man einen Festkörper, an dessen Oberfläche ein das Antigen spezifisch bindender Antikörper fixiert ist, in die flüssige Probe eintaucht und außerdem mit einem konjugierten Antikörper behandelt, der entweder direkt mit einem Farbstoff gelabelt oder mit einem in einer Nachreaktion einen Farbstoff bildenden Enzym konjugiert ist. Wenn sich das gesuchte Antigen in der flüssigen Probe befindet, wird dieses an den auf dem Festkörper fixierten Antikörper gebunden, während der konjugierte oder gelabelte weitere Antikörper, der sich im Überschuß befindet, andererseits an das Antigen gebunden wird. Auf diese Weise bekommt man auf dem Festkörper dann eine Farbstoffixierung oder -bildung, wenn das gesuchte Antigen in der Probe enthalten ist, andernfalls nicht.

Es sind auch schon verschiedene Vorrichtungen zur Ermittlung eines Antigens in einer flüssigen Probe bekannt, wie beispielsweise aus der US-PS 4 632 901. Diese Vorrichtung besteht aus einer Hülse, die mit einem porösen Absorbensmaterial gefüllt ist, über dem sich am oberen Ende der Hülse eine querliegende Membran anschließt, über der ein Trichterteil angeordnet ist. An die Membran ist der entsprechende Antikörper gebunden, der seinerseits das Antigen binden soll.

Bei der Benutzung dieser Vorrichtung wird die zu untersuchende Probe in das Trichterteil gegossen und wird von dem Absorbensmaterial durch die Membran hindurchgesaugt, wobei das gegenenfalls enthaltene Antigen an den an der Membran fixierten Antikörper gebunden wird.

Eine solche Vorrichtung ist allenfalls für Laboratorien geeignet, da ein bestimmtes Volumen der zu untersuchenden Probe in das Trichterteil einpipettiert werden muß, weil das untersuchte Probenvolumen nicht zu groß und nicht zu klein sein darf. Ein solches Pipettieren oder Volumenmessen kann im Heimgebrauch, beispielsweise bei Schwangerschaftstests, nicht durchgeführt werden, da die meisten Menschen im Umgang mit Einrichtungen, wie Pipetten, nicht geübt sind, so daß zu große Fehlergefahren mit solchen Methoden und Vorrichtungen verbunden sind.

Für den Heimgebrauch muß die Durchführung des Tests möglichst einfach und sicher ohne die Möglichkeit von Fehlerquellen sein.

Die der Erfindung zugrundeliegende Aufgabe bestand somit darin, ein Verfahren und eine Vorrichtung zur Ermittlung eines Antigens oder Antikörpers in einer flüssigen Probe zu bekommen, die besonders einfach und sicher durchzuführen sind und bei denen ungeübte Personen praktisch keine Fehler machen können. Insbesondere soll bei dem Verfahren ein Zupipettieren von Flüssigkeit oder eine andere Volumenmessung vermieden werden. Bevorzugt sollte sogar ein Umgießen der Probenlösung, wie Urin, vermieden werden.

Das erfindungsgemäße Verfahren zur Ermittlung eines Antigens oder Antikörpers in einer flüssigen Probe, bei dem man die Probe mit einer porösen Membran, an die ein entsprechender Antikörper bzw. entsprechendes Antigen gebunden ist und an die sich ein Flüssigkeit aufnehmendes poröses Absorbensmaterial anschließt, und einem sich an das Antigen bzw. den Antikörper bindenden konjugierten oder gelabelten Antikörper in Berührung bringt und gegebenenfalls diesen Antikörper unter Bildung eines Farbstoffes entwickelt ist dadurch gekennzeichnet, daß man in einem Behälter ein solches Volumen der flüssigen Probe, daß mit ihm das Porenvolumen des Absorbensmaterials nur teilweise füllbar ist, vorliegt, die Membran mit dem daran anschließenden Absorbensmaterial derart in die flüssige Probe eintaucht, daß diese im wesentlichen vollständig oder in einem vorbestimmten Volumen entgegen der Schwerkraft durch die Membran in das Absorbensmaterial gesaugt wird, und gegebenenfalls schließlich nach Entfernung der Membran aus der Probe sie in eine Entwicklerlösung einraucht und diese entgegen der Schwerkraft in das Absorbensmaterial einsaugen läßt.

Der konjugierte oder gelabelte Antikörper kann zusammen mit der flüssigen Probe oder anschließend an diese durch die Membran gesaugt werden. Hierzu ist er entweder in der flüssigen Probe bei deren Einsaugen enthalten oder befindet sich in einer separaten Lösung, die nach der flüssigen Probe eingesaugt wird.

Es ist wesentlich, das Absorbensvolumen und das einzusaugende Volumen der flüssigen Probe so aufeinander abzustimmen, daß beim Einsaugen der flüssigen Probe die Poren des Absorbensmaterials nur teilweise gefüllt werden. Der Grund ist der, daß das Absorbensmaterial genügende Saugkraft behalten muß, bis das erwünschte Volumen der flüssigen Probe eingesaugt ist. Dies wäre nicht der Fall, wenn die Poren vorzeitig mit Flüssigkeit gefüllt wären. Außerdem muß das Absorbensmaterial gegebenenfalls noch Saugkapazität für anschließendes Einsaugen der Lösung des konjugierten oder gelabelten Antikörpers oder der Entwicklerlösung behalten.

Mit dem erfindungsgemäßen Verfahren kann man also sowohl Antigene als auch Antikörper in der flüssigen Probe ermitteln. Im ersteren Fall ist an die Membran ein Antikörper gebunden, an den sich das gesuchte Antigen bindet, an welches sich seinerseits der konjugierte oder gelabelte zweite Antikörper bindet. Im letzteren Fall ist an die Membran ein Antigen gebunden, an welches sich der gesuchte Antikörper bindet. Der konjugierte oder gelabelte zweite Antikörper bindet sich dann seinerseits an den an das Antigen gebundenen ersten Antikörper.

Bevorzugt verwendet man als konjugierten Antikörper einen mit einem Farbstoffbildner konjugierten Antikörper, wie einen solchen, der mit einem in einer Nachreaktion einen Farbstoff bildenden Enzym oder einer durch nachfolgende Oxidation oder Reduktion einen Farbstoff bildenden Gruppe konjugiert ist. Durch Vermeidung eines direkt mit Farbstoff gelabelten Antikörpers erreicht man, daß in der Nachreaktion oder Entwicklung ausschließlich dann und dort Farbstoff gebildet wird, wo der konjugierte Antikörper über das Antigen und den fixierten Antikörper bzw. über den ersten Antikörper und das fixierte Antigen an die Membran gebunden ist, und nicht anderweitig Farbstoff an der Membran adsorbiert ist, was das Ergebniss verfälschen könnte.

Die Nachreaktion oder Entwicklung des Farbstoffes ist ebenso einfach wie die Bindung des Antigens auf der Membran und bedarf einfach eines Eintauchens der Membran mit dem anschließenden Absorbensmaterial in die Entwicklerlösung. Dabei sollte das Absorbensmaterial noch ausreichend freie Poren haben, um die Entwicklerlösung durch die Membran saugen zu können, was bedeutet, daß in der ersten Arbeitsstufe nur ein Teil des Porenvolumens des Absorbensmaterials mit der flüssigen Probe gefüllt werden darf.

Im allgemeinen dürfte es zweckmäßig sein, zwischen dem Einsaugen der flüssigen Probe zur Fixierung des Antigens über den ersten Antikörper bzw. des ersten Antikörpers über das Antigen auf der Membran und vor dem Einsaugen der Entwicklerlösung die Membran von überschüssigem Probenmaterial und/oder konjugiertem oder gelabeltem Antikörper freizuspülen. Dies kann beispielsweise unter fließendem Wasser oder durch Eintauchen in und Einsaugen von Spülwasser geschehen.

Weiterhin ist es zweckmäßig, die flüssige Probe vor dem Einsaugen durch die Membran zu filtrieren, um feste Bestandteile daraus zu entfernen, die die Poren der Membran verstopfen könnten.

Im vorliegenden Verfahren ist es von Bedeutung, das Probenvolumen in etwa vorzubestimmen, um einerseits zu vermeiden, daß das gesamten Porenvolumen des Absorbensmaterials mit der Probe gefüllt wird und um andererseits das Einsaugen eines zu geringen Porenvolumens zu vermeiden, da dieses üblicherweise zu wenig Antigen bzw. Antikörper auf der Membran binden und damit eine zu geringe Farbreaktion ergeben würde.

Das erfindungsgemäße Verfahren ist äußerst einfach durchzuführen und schließt praktisch auch für ungeübte Personen Fehlerquellen aus.

Mit einem sich bis zum Rand füllenden kleinen Meßbecher, der beispielsweise für einen Schwangerschaftstests in den Urinstrahl gehalten wird, wird ohne Fehlermöglichkeit ein bestimmtes Flüssigkeitsvolumen abgemessen und in einen Probenbehälter überführt, in dem der Test gemacht wird. Stattdessen kann der Probenbehälter auch selbst als Meßbecher ausgebildet sein und in den Urinstrahl gehalten werden. Es erübrigt sich dann das Umkippen aus dem Meßbecher in den Probenbehälter, was seinerseits eine geringe Fehlerquelle durch Verschütten beinhaltet.

In beiden Fällen wird dann der Absorbensmaterial-Behälter mit der Membran am Ende in den Probenbehälter eingetaucht, worauf die Probenlösung durch die Membran in das Absorbensmaterial gesaugt wird. Wenn ein bestimmtes Volumen der Probe oder vorzugsweise im wesentlichen die gesamte Probe eingesaugt ist, wird der Absorbensmaterial-Behälter herausgenommen, gegebenenfalls gespült und anschließend gegebenenfalls in die Entwicklerlösung eingetaucht. Nach einer bestimmten Zeit wird der Absorbensmaterial-Behälter herausgenommen und die Membran visuell hinsichtlich der Farbreaktion geprüft

Wenn hier von einem vorbestimmten Volumen der flüssigen Probe die Rede ist, so braucht dieses nicht genau eingehalten zu werden, sondern kann beispielsweise um 20 oder 30 % schwanken, ohne das qualitative Ergebnis zu beeinträchtigen.

Die Einfachheit und Sicherheit der Testdurchführung erlaubt es, das Verfahren und die Vorrichtung nach der Erfindung für den Heimgebrauch zu verwenden. Die Vorteile sind folgende: Es entfällt ein Einpipettieren der Probenflüssigkeit. Es erübrigt sich, die Probenflüssigkeit in einem Trichter auf die Membran aufzugießen, was die Gefahr eines Verschüttens beinhaltet. Durch einfaches Eintauchen der Membran erfolgt ein selbsttätiger Durchtritt der Probe durch die Membran ohne die Gefahr eines Verschüttens. Selbst wenn der Test etwa durch Abrufen der Testperson durch ein Telefongespräch oder dergleichen stehenbleibt, erfolgt kein Austrocknen der Membran, was für das Ergebnis schädlich wäre und beispielsweise bei Vorrichtungen gemäß der US-PS 4 632 901 möglich ist.

Durch die kleine Membranfläche und ein verhältnismäßig großes Durchtrittsvolumen bekommt man eine potentiell hohe Empfindlichkeit. Infolge der kleinen Membranfläche bekommt man geringe Reagenzkosten, da nur wenig erster auf der Membran fixierter Antikörper benötigt wird.

Anzumerken ist noch, daß der zweite konjugierte oder gelabelte Antikörper im Überschuß gegenüber dem ersten Antikörper bzw. daran zu bindenden Antigen vorliegt und in dem Probenbehälter untergebracht ist, so daß sich der zweite konjugierte oder gelabelte Antikörper beim Eingießen der Probe mit dieser vermischt.

Nach einer besonders zweckmäßigen Ausführungsform wird die erfindungsgemäß verwendete Membran gleichzeitig mit einer Kontrollmöglichkeit versehen, mittels derer gleichzeitig festgestellt werden kann, ob der Test noch in Ordnung oder etwa durch überlange Lagerung oder dergleichen unbrauchbar ist. Hierzu wird der erste Antikörper bzw. das Antigen nur auf einem Teil der Membran gebunden, während auf einem anderen Teil entweder das spezifische Antigen oder ein anderer den zweiten, konjugierten oder gelabelten Antikörper bindender Antikörper fixiert wird. In beiden Fällen wird, wenn der Test noch in Ordnung ist, der im Überschuß in der Probe vorliegende konjugierte oder gelabelte zweite Antikörper gebunden. WEnn an der Membran nicht ein Antigen, sondern ein vom zweiten Antikörper verschiedener Antikörper zur Bindung des zweiten Antikörpers fixiert ist, kann diese Fixierung direkt über den zweiten Antikörper oder über das an ihn gebundene Enzym erfolgen.

Die für das erfindungsgemäße Verfahren verwendete Vorrichtung unterscheidet sich ebenfalls wesentlich von anderen nach dem Stand der Tech-nik verwendeten Vorrichtungen. Die erfindungsge-mäße Vorrichtung mit einem porösen Membranteil, an das ein sich an das Antigen bindender Antikörper gebunden ist, und einer sich an das Membranteil anschließenden Säule eines porösen Absorbensmaterials ist gekennzeichnet durch einen Probenbehälter und einen rohrförmigen Absorbensmaterial-Behälter, der letzterer an einem Ende durch das Membranteil verschlossen und am anderen Ende offen ist, wobei Probenbehälter und Absorbensmaterial-Behälter so bemessen sind, daß der Absorbensmaterial-Behälter mit der Membran nach unten in den Probenbehälter eintauchbar ist.

Wenn bei dieser Vorrichtung eine von dem Absorbensmaterial getrennte Membran verwendet wird, ist diese aufgrund ihrer geringen Dicke und Flexibilität in der Gefahr, beim Eindrücken des Absorbensmaterials nach außen gebogen zu werden. Deswegen ist es zweckmäßig, zwischen dem Absorbensmaterial und der Membran einen porösen starren Stützkorper anzuordnen. Das Absorbensmaterial kann dabei stückig sein oder aus einer einstückigen Säule bestehen.

Bevorzugt ist es, wenn das Absorbensmaterial aus einer Säule besteht und einstückig mit dem Membranteil verbunden ist. Solche Absorbensmaterialien bestehen beispielsweise aus einem porigen Kunststoffstab, der an seiner einen Stirnseite einstückig mit einer porösen Membran aus einem Cellulosematerial oder dergleichen verbunden ist.

Wesentlich ist, daß das Absorbensmaterial von einer flüssigkeitsundurchlässigen Hülle umgeben ist, die gewährleistet, daß Flüssigkeit in das Absorbensmaterial ausschließlich durch die Membran eingesaugt wird. Dabei kann es sich um einen rohrförmigen Metallbehälter oder um eine die Ab-sorbensmaterialsäule umgebende flüssigkeitsun-durchlässige Kunststoffhaut handeln.

Besonders in letzterem Falle kann der rohrförmige Absorbensmaterial-Behälter auch zu mehreren aneinanderhängend in den Han del gebracht und im Bedarfsfall einzeln abgerissen oder abgebrochen werden, und hierzu ist es zweckmäßig, zwischen den einzelnen zusammenhängenden Absorbensmaterial-Behältern Bruchlinien oder Schwächungslinien vorzusehen, die ein leichtes Abreißen, etwa durch seitliches Umbiegen, ermöglichen.

Bei einer anderen Ausführungsform der Erfindung können auch mehrere der Absorbensmaterial-Behälter fest miteinander verbunden sein, um etwa in Laboratorien oder Arztpraxen Reihentests durch-zuführen. Diese Mehrfachbehälter können bei-spielsweise in Verbindung mit Verdünnungstest-platten benutzt werden, wobei die Vertiefungen in den Verdünnungstestplatten als Probenbehälter dienen. Mit einer solchen Vorrichtung können gleichzeitig mehrere Tests auf einmal gemacht werden, da die zusammenhängenden Absorbensmaterial-Behälter gleichzeitig in mehrere Vertiefungen der Verdünnungstestplatten eingetaucht werden.

Da es zweckmäßig ist, zur Vermeidung einer Verstopfung der Membranporen feste Teilchen aus der Probe zu entfernen, bevor diese mit dem Membran in Berührung kommt, ist es bevorzugt, dem Membranteil in Strömungsrichtung der Probe entgegen der Schwerkraft ein Filter vorzuschalten. Dieses kann eine Filterplatte sein, die unmittelbar vor der Membran liegt und an dem Absorbensmaterial-Behälter befestigt ist. Der Filter kann aber auch an geeigneter Stelle in dem Probenbehälter derart eingebaut sein, daß die gesamte flüssige Probe den Filter passieren muß, bevor sie zu der Membran gelangt.

Eine bevorzugte Vorrichtung nach der Erfindung besitzt einen Außenbehälter, einen in den Außenbehälter einsetzbaren Probenbehälter, einen an einem Ende durch das Membranteil verschlossenen, am anderen Ende mit Öffnung versehenen rohrförmigen Absorbensmaterial-Behälter, der in den Probenbehälter einsetzbar und an einem auf dem Außenbehälter aufsetzbaren Halteteil befestigt ist, sowie einen in das Halteteil und einen Fortsatz des Probenbehälters eingreifenden, auf den Außen-behälter aufsetzbaren, abnehmbaren Abstandshal-ter, der den Fortsatz des Probenbehälters mit den Halteteil lösbar verbindet, wobei die Abmessungen von Außenbehälter, Abstandshalter und Absorbensmaterial-Behälter derart sind, daß das Membranteil bei aufgesetztem Abstandshalter oberhalb des Probenbehälters, bei abgenommenem Abstandshalter in dem Probenbehälter angeordnet ist.

Vorzugsweise ist der Abstandshalter seitlich abnehmbar und zu diesem Zweck im wesentlichen U-förmig ausgebildet, wobei er mit einer ersten Nut oder Feder in eine Feder bzw. Nut am Halteteil und mit einer zweiten Nut oder Feder in eine Feder bzw. Nut am oberen Bereich des Fortsatzes des Probenbehälters eingreift. Dabei kann die jeweils in eine Nut eingreifende Feder unterschiedlich ausgebildet sein, wie beispielsweise in Form eine die Nut im wesentlichen ganz ausfüllenden Rippe oder in Form einzelner Vorsprünge, die beispielsweise punktförmig auf einer in die Nut eingreifenden Linie angeordnet sein können.

Die lösbare Verbindung des Abstandshalters mit dem Fortsatz des Probenbehälters erfolgt in einfacher Weise dadurch, daß der Fortsatz des Probenbehälters am oberen Ende eine Wulst aufweist, die die Feder bildet und in eine Nut des vorzugsweise U-förmigen Abstandshalters einschiebbar ist. Umgekehrt kann selbstverständlich auch der Abstandshalter eine Rippe aufweisen, die in eine Nut am oberen Ende des Fortsatzes des Probenbehälters eingreift. Den gleichen Effekt bekommt man auch, wenn der Fortsatz des Probenbehälters am oberen Ende eine Wulst besitzt, die auf einer Schulter an der Innenseite des Abstandshalters aufliegt.

Wesentlich bei allen diesen möglichen Ausführungen ist es, daß bei aufgesetztem Abstandshalter das Halteteil mit dem Probenbehälter über dessen Fortsatz verbunden ist, während bei abgenommenen Halteteil automatisch diese Verbindung gelöst ist, so daß beim Abnehmen des Halteteils von dem Außenbehälter der Probenbehälter in dem Außenbehälter zurückbleibt.

Der Probenbehälter und sein Fortsatz können unterschiedlich ausgebildet sein. Zweckmäßig ist der Fortsatz des Probenbehälters im wesentlichen rohrförmig ausgebildet. Der Probenbehälter selbst ist dann günstigerweise ein unterer becherförmiger, d.h. am unteren Ende verschlossener, seitlich in einem bestimmten Abstand vom Becherboden mit Flüssigkeitseintrittsöffnungen versehener Endabschnitt dieses rohrförmigen Fortsatzes. Die seitlichen Flüssigkeitseintrittsöffnungen bilden ein Mittel zur Bestimmung des Flüssigkeitsspiegels in dem Probenbehälter, da bei seitlichem Einführen der Flüssigkeit sich der Probenbehälter nur bis zu diesen seitlichen Flüssigkeitseintrittsöffnungen füllt. Damit ist gewährleistet, daß der Probenbehälter nur eine vorbestimmte Flüssigkeitsmenge aufnimmt.

Das Halteteil, das dazu bestimmt ist, den Probenbehälter bei Verbindung mit ihm über den Abstandshalter aus dem Außenbehälter zu nehmen, ist vorzugsweise als den Außenbehälter verschließender Deckel ausgebildet, um während des Stehens der Vorrichtung beispielsweise das Hineinfallen von Fremdsubstanzen zu vermeiden. An sich reicht es aus, daß das Halteteil und der Abstandshalter in irgendeiner Weise auf dem Außenbehälter aufsetzbar sind. Zweckmäßig ist es jedoch, daß der untere Rand des Halteteils und des Abstandshalters derart ausgebildet sind, daß beide Teile alternativ auf die Öffnung des Außenbehälters aufgepaßt sind.

Die Funktionsweise der neuerungsgemäßen Vorrichtung ist folge

Zu Beginn ist die Vorrichtung derart zusammengesetzt, daß sich der Abstandshalter in aufgesetztem Zustand zwischen dem Außenbehälter und dem Halteteil befindet, was zur Folge hat, daß der Probenbehälter mit dem Halteteil verbunden ist und mit diesem aus dem Außenbehälter herausnehmbar ist.

Bei Verwendung der Vorrichtung für einen Test wird das Halteteil mit dem über den Abstandshalter daran befestigten Probenbehälter aus dem Außenbehälter herausgenommen, worauf die Öffnungen des Probenbehälters in den Urinstrahl gehalten werden, bis der Probenbehälter in der vorbestimmten Höhe mit Urin gefüllt ist.

Hierfür ist es zweckmäßig, daß der rohrförmige Fortsatz des Probenbehälters auf seinem Umfang zwischen den seitlichen Flüssigkeitseintrittsöffnungen mit längs verlaufenden Ablaufrippen versehen ist. Dies erleichtert die sichere Funktion der Vorrichtung. Wenn nämlich nun einfach der rohrförmige mit Ablaufrippen versehene Fortsatz des Probenbehälters in den Urinstrahl gehalten wird, läuft der Urin entlang den von den Ablaufrippen gebildeten Rinnen automatisch in die seitlichen Flüssigkeitseintrittsöffnungen, bis der Flüssigkeitsspiegel diese Flüssigkeitseintrittsöffnungen erreicht, welche dann als Überlauf dienen.

Die Vorrichtung ist so zusammengesetzt, daß der an dem Halteteil befestigte rohrförmige Absorbensmaterial-Behälter in dem rohrförmigen Fortsatz des Probenbehälters eingesetzt ist. In dem beschriebenen Zustand mit aufgesetztem Abstandshalter liegt jedoch das untere Ende des Absorbensmaterial-Behälters, d. h. das Membranteil, oberhalb des Probenbehälters oder mit anderen Worten oberhalb der seitlichen Flüssigkeitseintrittsöffnungen und taucht daher in diesem Zustand nicht in den Urin in den Probenbehälter ein.

Wenn der Probenbehälter mit Urin gefüllt ist, wird der Abstandshalter mit daran befestigtem Fortsatz des Probenbehälters und Halteteil wieder auf den Außenbehälter aufgesetzt. Hierauf wird der Abstandshalter abgenommen, d. h. in der bevorzugten U-förmigen Ausführungsform seitlich abgezogen. Dabei löst sich der Fortsatz des Probenbehälters von dem Halteteil, und gleichzeitig rutscht der Absorbensmaterial-Behälter mit dem daran befestigten Halteteil über den Abstand des Abstandshalters tiefer in den Fortsatz des Probenbehälters hinein, bis die Membran in dem Probenbehälter angeordnet ist.

In diesem Zustand wird infolge der Saugwirkung des Absorbensmaterials in dem Absorbensmaterial-Behälter die Urinprobe in dem Probenbehälter durch das Membranteil in das Absorbensmaterial darüber gesaugt, wobei sich das gesuchte Antigen in der Urin probe an den an das Membranteil gebundenen Antikörper bzw. der gesuchte Antikörper in der Urinprobe an das an das Membranteil gebundene Antigen bindet.

Für eine Nachreaktion oder Entwicklung des Farbstoffes ist es bevorzugt, daß der Außenbehälter zusätzlich zu den oben beschriebenen Teilen einen Entwicklerbehälter enthält, in den der Absorbensmaterial-Behälter einfügbar ist. Der Entwicklerbehälter kann ein Abteil des Außenbehälters sein oder beispielsweise ein an dem Außenbehälter befestigter rohrförmiger Innenbehälter sein, der so dimensioniert ist, daß er den Absorbensmaterial-Behälter aufnehmen kann.

Bei dem Test geht man nach Beendigung der oben beschriebenen Stufe so vor, daß man nach dem Einsaugen der Urinprobe durch das Membranteil in den Absorbensmaterial-Behälter das Halteteil mit dem daran befestigten Absorbensmaterial-Behälter abnimmt und so verdreht, daß der Absorbensmaterial-Behälter in den Entwicklerbehälter eingetaucht wird. Nach der für die Nachreaktion oder Entwicklung erforderlichen Eintauchzeit wird der Absorbensmaterial-Behälter aus dem Entwicklerbehälter mit Hilfe des Halteteils herausgenommen, worauf visuell festgestellt wird, ob sich auf dem Membranteil ein Farbstoff gebildet hat, der nun ein positives Testergebnis anzeigt.

Durch die Zeichnung wird die Erfindung weiter erläutert. In dieser bedeuten

Figur 1 einen senkrechten Schnitt durch eine erste Ausführungsform einer Vorrichtung nach der Erfindung,

Figur 2 einen senkrechten Schnitt durch eine zweite Ausführungsform einer Vorrichtung nach der Erfindung, und

Figur 3 eine Draufsicht auf eine bevorzugte Ausführungsform eines Absorbensmaterial-Behälters von unten,

Figur 4 eine perspektivische Darstellung einer Ausführungsform der neuerungsgemäßen Vorrichtung in zusammengesetztem Zustand mit aufgesetztem Abstandshalter,

Figur 5 eine perspektivische Darstellung der in Figur 4 gezeigten Ausführungsform nach Entfernung des Abstandshalters und mit herausgezogenem Absorbensmaterial-Behälter und

Figur 6 eine Explosionsdarstellung der in Figur 4 und 5 gezeigten Vorrichtung mit den verschiedenen Einzelteilen in perspektivischer Darstellung.

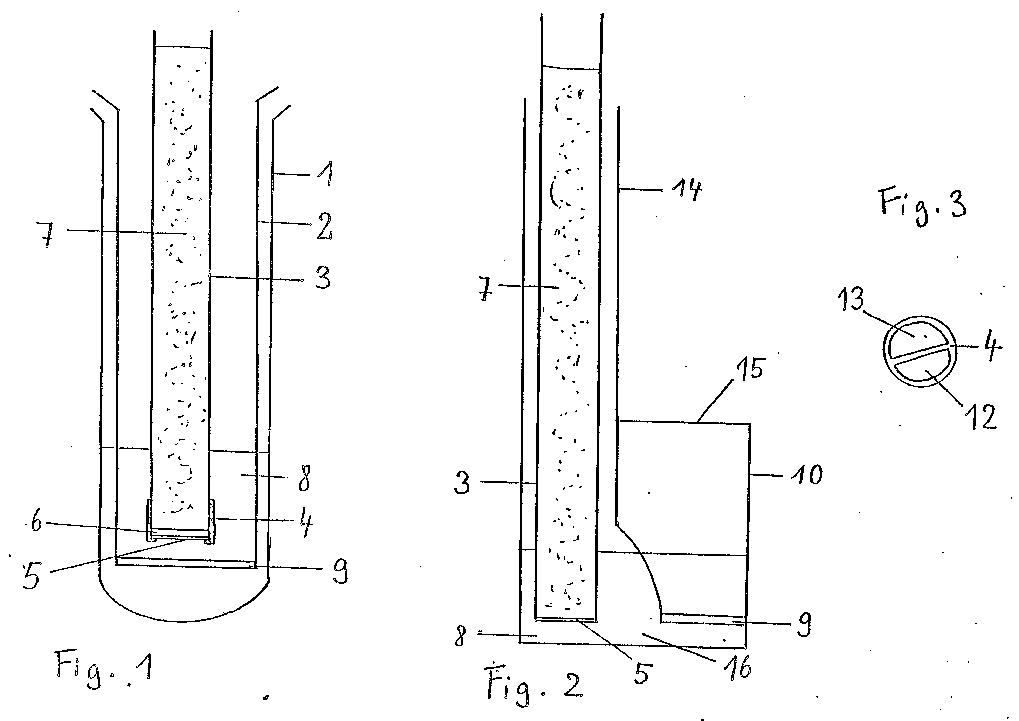

Die in Fig. 1 dargestellte Ausführungsform einer Vorrichtung nach der Erfindung zeigt einen Probenbehälter 1 etwa in der Form eines Reagenzglases. In diesem ist konzentrisch ein zweiter Behälter 2 eingesetzt, der am unteren Ende von einem Filter 9 verschlossen ist. In dem Behälter 2 ist der Absorbensmaterial-Behälter 3 eingesetzt, der mit Absorbensmaterial 7 gefüllt ist. Das untere Ende des Absorbensmaterial-Behälters 3 ist von einer Kappe 4 verschlossen, die eine Membran 5 haltert. Zwischen der Membran 5 und dem Absorbensmaterial 7 ist ein poröses starres Stützteil 6 angeordnet.

Bei Benutzung dieser Testvorrichtung gemäß Fig. 1 wird zunächst vor dem Einsetzen des Behälters 2 und des Absorbensmaterial-Behälters 3 mit einem kleinen Meßbecher Probenflüssigkeit 8 eingegossen. Daraufhin wird der Behälter 2 mit dem Filter 9 am unteren Ende eingesetzt, wobei die Probenflüssigkeit durch den Filter 9 in den Behälter 2 hingedrückt wird. Daraufhin wird der Absorbensmaterial-Behälter 3 mit der Kappe 4 nach unten eingesetzt. Daraufhin wird automatisch Flüssigkeit 8 durch die Membran 5 hindurch in das Absorbensmaterial 7 gesaugt. Wenn die Flüssigkeit 8 bis zur Höhe des Filters 5 eingesaugt ist, bleibt noch genügend Kapazität im Porenvolumen des Absorbensmaterials 7, um bei der Überführung des Absorbensmaterial-Behälters 3 in eine Entwicklerflüssigkeit von dieser in ausreichender Menge in das Absorbensmaterial einzusaugen.

Die in Fig. 2 dargestellte Ausführungsform einer Vorrichtung nach der Erfindung ist bevorzugt, da kein zusätzlicher Meßbecher und kein Umgießen der Probenflüssigkeit erforderlich ist.

Der in Fig. 2 dargestellte Probenbehälter 10 besitzt einen rohrförmigen Ansatz 14, der gleichzeitig als Handgriff verwendet wird, um den Probenbehälter 10 mit der Öffnung 15 in den Urinstrahl zu halten. Der Probenbehälter 10 läuft über, wenn der Raum zwischen der Öffnung 15 und dem Filter 9, der Außenwandung und der Trennwand 11 mit der Probenflüssigkeit gefüllt ist. Dieser definierte Raum begrenzt daher das im Test erwünschte Volumen der Probenflüssigkeit.

Bei kurzem Stehen läuft dann die Probenflüssigkeit so weit durch den Filter 9 in den Raum 16, bis die Flüssigkeit 8 den in der Zeichnung gezeigten Flüssigkeitsstand erreicht hat.

Sodann wird der Absorbensmaterial-Behälter 3 mit der Membran 5 nach unten in den rohrförmigen Ansatz 14 eingeführt, worauf die Flüssigkeit bis zur Höhe der Membran in das Absorbensmaterial 7 eingesaugt wird.

Bei der in Fig. 2 dargestellten bevorzugten Ausführungsform besteht das Absorbensmaterial 7 aus einer von Poren durchsetzten Kunststoffsäule, die an ihrem unteren Ende einstückig mit der Membran 5 verbunden ist und die von einer flüssigkeitsundurchlässigen Stoffhaut umgeben ist.

In Fig. 3 ist eine bevorzugte Ausführungsform der in Fig. 1 wiedergegebenen Kappe 4 des Absorbensmaterial-Behälters wiedergegeben. Diese Kappe besitzt zwei voneinander getrennte Eintrittsöffnungen über der Membran, wobei der Membranteil 12 den fixierten Antikörper trägt, der für das gesuchte Antigen spezifisch ist, während der Membranteil 13 fixiertes Antigen oder einen vom konjugierten oder gelabelten zweiten Antikörper verschiedenen Antikörper trägt, wobei sowohl das Antigen als auch der andere Antikörper in der Lage sind, den zweiten Antikörper zu binden. Dieses Teil 13 der Membran dient der Kontrolle der Funktionsfähigkeit des Tests.

Selbstverständlich kommt der Probenbehälter 1 bzw. 10 den konjugierten oder gelabelten zweiten Antikörper enthaltend in den Handel, so daß sich dieser zweite Antiköper beim Eingießen der Probe automatisch oder gegebenenfalls bei kurzem Schütteln mit der Probenflüssigkeit vermischt.

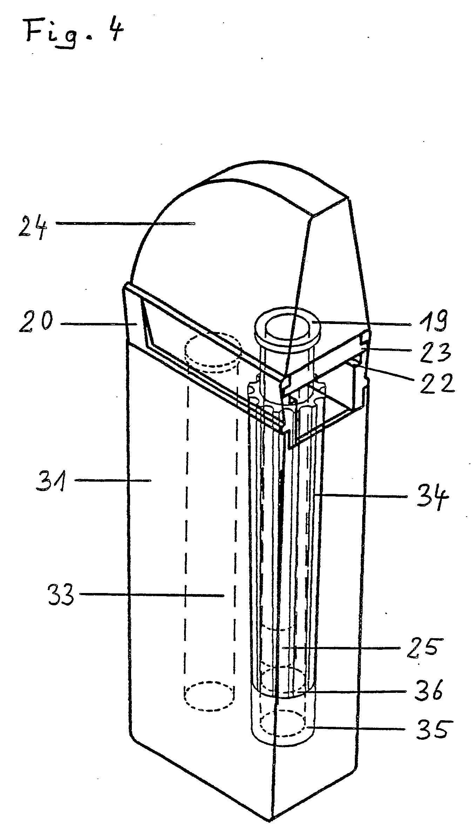

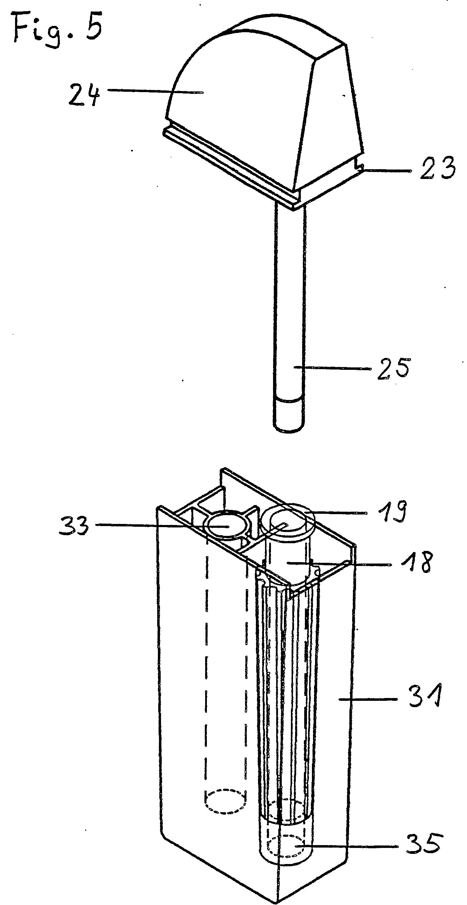

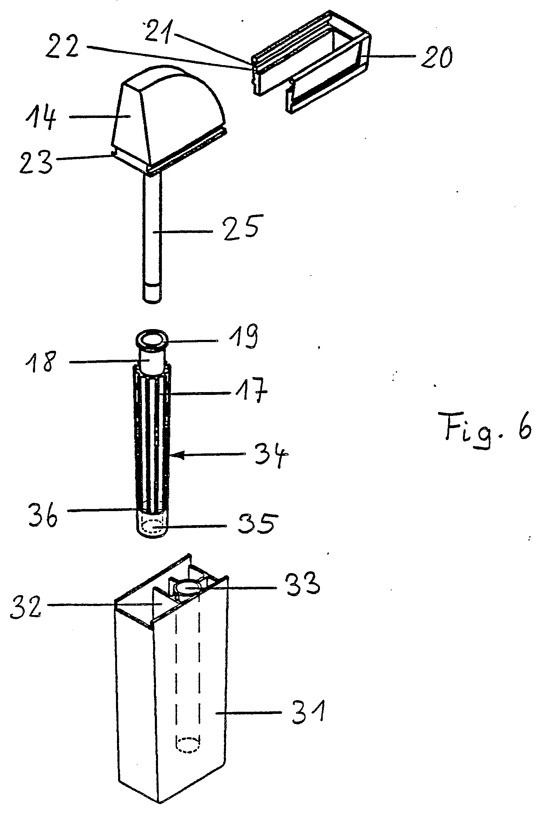

Die in den Figuren 4 bis 6 dargestellte Vorrichtung besteht aus einem Außenbehälter 31 mit zwei durch eine Trennwand 32 voneinander getrennten Abteilen, von denen eines leer ist und das andere einen darin befestigten Entwicklerbehälter 33 in Form eines unten geschlossenen Rohres enthält. Weiterhin enthält die Vorrichtung ein Teil 34, das am unteren Ende den becherförmigen Probenbehälter 35 mit einem Kranz seitlicher Flüssigkeitseintrittsöffnungen 36 zwischen in Längsrichtung des an den Probenbehälter 35 anschließenden Fortsatzes 18 verlaufenden Ablaufrippen 17 aufweist. Am oberen Ende besitzt der Fortsatz 18 eine Ringwulst 19, die über einer Schulter des Abstandshalters 20 einschiebbar ist.

Der Abstandshalter 20 besitzt im wesentlichen U-Form und ist mit seinem unteren Ende auf die Öffnung des Außenbehälters 31 aufgepaßt. Im oberen Bereich besitzt der Abstandshalter 20 auf beiden Innenseiten Nuten 21, in die die beiderseitigen Rippen 23 des Halteteils 24 einschiebbar sind. Außerdem besitzt der Abstandshalter 20 unter den Nuten 21 jeweils eine Schulter 22, über der die Ringwulst 19 des Fortsatzes 18 einschiebbar ist.

An dem Halteteil 24 ist der Absorbensmaterial-Behälter 25 befestigt. Dieser ist am unteren Ende durch eine durchlässige Membran verschlossen, besitzt am oberen, nicht gezeigten Ende Entlüftungsöffnungen und ist mit einem saugfähigen Absorbensmaterial gefüllt.

Figur 1 zeigt die Vorrichtung in dem zusammengebauten Anfangszustand, in welchem der U-förmige Abstandshalter 20 zwischen das Halteteil 24 und den Außenbehälter 31 eingeschoben ist und durch eine Nut-Feder-Verbindung das Halteteil und durch die Auflage der Ringwulst 19 des Fortsatzes 18 auf der Schulter 22 den Probenbehälter an sich fixiert. In diesem Zustand liegt das untere Ende des Absorbensmaterial-Behälters 25 mit dem Membranteil oberhalb des Kranzes von Flüssigkeitseintrittsöffnungen 36 und damit oberhalb des Probenbehälters 35. In diesem Zustand kann also das Halteteil 24 zusammen mit dem Abstandshalter 20 und dem Probenbehälter 5 und dessen Fortsatz 18 vom Außenbehälter 31 abgenommen werden, um den Probenbehälter in den Urinstrahl zu halten.

Wie Figur 2 zeigt, löst sich durch Abnahme des Abstandshalters 20 der Fortsatz 18 des Probenbehälters 35 und bleibt beim Herausnehmen des Halteteils 24 mit dem Absorbensmaterial-Behälter 25 in dem Außenbehälter 31. Beim Aufsetzen des Halteteils 24 auf den oberen Rand des Außenbehälters 31 rutscht das untere Ende des Absorbensmaterial-Behälters 25 mit dem Membranteil bis in den Probenbehälter 35 und kann daher die in diesem Probenbehälter 35 enthaltene Urinprobe durch die Membran aufsaugen.

Anschließend hieran wird das Halteteil, wie in Figur 2 dargestellt, erneut abgenommen, um 180° gedreht und sodann in den Entwicklerbehälter 33 eingetaucht, in dem das Halteteil in umgekehrter Richtung auf den Außenbehälter 31 aufgesetzt wird. Nach Ablauf der Entwicklungszeit wird das Halteteil 24 mit dem daran befestigten Absorbensmaterial-Behälter 25 aus dem Entwicklerbehälter 33 herausgezogen und das Testergebnis auf dem Membranteil visuell ermittelt.

Beispiel 1

In ein rundes Gefäß, dessen Innendurchmesser 2 mm größer ist als der Außendurchmesser des Absorbensmaterial-Behälters, werden 0,2 ml Urin pipettiert, der mit zweitem Antikörper gegen HCG [1], gelabelt mit alkalischer Phosphatase, gemischt ist und der jeweils 0,50, 100, 200 I.U. HCG/1 enthält. Vor das Absorbensmaterial ist eine Membran mit der Porengröße 3 μ plaziert, die mit erstem Antikörper gegen HCG auf einer Hälfte, mit HCG auf der anderen Hälfte beschichtet wurde.

Der                    Absorbensmaterial-Behälter

(1) HCG  = Human-Chorion-Gonadotropin

(Außendurchmesser 11 mm) wird in den Urin eingetaucht und darin belassen, bis das gesamte Volumen durchgesaugt ist (ca. 30 sec). Danach wird der Absorbensmaterial-Behälter in ein zweites rundes Gefäß überführt, das eine Lösung von 3 mMol/l Indoxylphosphat in 1 M Diethanolamin, pH 9,8, enthält (0,5 ml).

Der Absorbensmaterial-Behälter wird ebenfalls hierin belassen, bis das gesamte Volumen der zweiten Lösung, die gleichzeitig als Waschlösung und als Substrat für alkalische Phosphatase dient, durch die Membran gesaugt wurde (ca. 1 min).

Folgende Resultate wurden erhalten:

Die Hälfte der Membran, die mit HCG beschichtet ist, zeigt nach Entnahme des Absorbensbehälters aus dem zweiten Gefäß eine dunkelblaue Färbung bei jeder im Experiment verwendeten Urinlösung. Die andere Hälfte, die mit Antikörper gegen HCG beschichtet ist, zeigt bei dem Urin, der kein HCG enthält, einen weißen Hintergrund, der sich im Laufe der Zeit leicht bläulich verfärbt, während bei den HCG enthaltenden Urinen eine deutliche, mit zunehmender Konzentration intensiver blau werdende Färbung sichtbar ist.

Durch kurzes Waschen der Membran in fließendem Leitungswasser (5 bis 10 sec) direkt nach Entnahme aus der zweiten Lösung kann die leichte Blauverfärbung, die sich beim Urin ohne HCG ergab, verhindert werden.

## Beispiel 2

Die Durchführung geschieht analog zu Beispiel 1 mit dem Unterschied, daß zuerst der Urin aufgesaugt (0,2 ml) wird, anschließend in einem zweiten Gefäß der gelabelte Antikörper aufgesaugt wird (0,2 ml) und dann die Substrat- und Waschlösung aufgesaugt wird (0,5 ml). Die Ergebnisse sind identisch, wobei die in diesem Beispiel angeführte Durchführungsweise den Vorteil hat, daß ein positives Ergebnis auch noch bei sehr hohen HCG-Konzentrationen (über 200 000 I.U. HCG/l) erhalten wird, ohne daß sehr hohe Mengen an zweitem Antikörper eingesetzt werden müssen.

## Beispiel 3

Die Durchführungsweise geschieht analog zu Beispiel 1, wobei der erste Antikörper und HCG kreuzweise auf die Membran aufgebracht werden, dergestalt, daß der erste Antikörper einen Balken und HCG den anderen Balken des Kreuzes darstellt. Bei dieser Gestaltung zeigt bei nicht HCG enthaltenden Urinen ein Balken eine Blaufärbung, nämlich der aus HCG bestehende, was als negativ oder minus zu interpretieren ist, während bei HCG enthaltenden Urinen beide Balken eine Blaufärbung zeigen, was als positiv oder plus deutlich sichtbar wird. Gerade im Heimgebrauch ist diese Version als vorteilhaft anzusehen, da sie leicht und eindeutig abzulesen ist.

## Beispiel 4

Die Durchführungsweise geschieht analog zu Beispiel 2, wobei ein Teil der Membran mit Rubella [4]-Antigen und ein anderer Teil mit aus Kaninchen-Antikörpern, gerichtet gegen IgG [2] aus Schafen, beschichtet ist. Es werden zunächst Humanseren (0,2 ml) in Probenbehälter pipettiert und anschließend der Absorbensbehälter in die Proben eingetaucht, bis die Gesamtmenge des Serums durchgesaugt ist (ca. 1 min).

Dann werden die Absorbensbehälter in ein zweites Gefäß überführt, das Antikörper aus Schafen, gerichtet gegen humanes IgG und gelabelt mit alkalischer Phosphatase, enthält (0,2 ml). Die Gesamtmenge wird durchgesaugt (ca. 1 min), dann wird in ein drittes Gefäß überführt, das gepufferte Kochsalzlösung (0,9 %) mit 0,2 % Tween 20 [3], enthält (0,5 ml Dauer ca. 90 sec).

Anschließend wird in einem vierten Gefäß Substratlösung durchgesaugt (0,5 ml, Dauer ca. 2 min).

Die entstandene Färbung kann anschließend entweder durch Spülen mit Leitungswasser, wie im Beispiel 1 beschrieben, oder durch Überführen in ein weiteres Gefäß, das wieder gepufferte Kochsalzlösung mit 0,2 % Tween 20 enthält, stabilisiert werden.

Alle Seren zeigen im mit Kaninchen-Antikörper, gerichtet gegen Schafs-IgG, beschichteten Feld eine Blaufärbung, die durch die aufgebrachte Menge des Kaninchen-Antikörpers regulierbar ist und die so eingestellt wurde, daß die entstehende Blaufärbung der Menge an Antikörpern im Humanse-

(4) Rubella = das Röteln-Virus

(2) IgG = Immunglobulin G

(3) Tween 20 = Nichtionisches Detergenz, Markenname

rum entspricht, die im mit Rubella-Antigen beschichteten Feld dann auftritt, wenn das Humanserum eine ausreichende Immunität gegen Rubella aufweist (in der Regel ein Titer von 1 : 8 bis 1 : 16 im Hämagglutinationshemmtest, der als Referenzmethode dient).

Durch Vergleich der beiden Felder kann leicht festgestellt werden, ob der Untersuchte keine ausreichende Immunität aufweist (Blaufärbung im Rubella-beschichteten Feld geringer als im Kaninchen-Antikörper-beschichteten Feld) oder die Immunität gegeben ist (Blaufärbung gleich stark oder stärker).

Es kann leicht gesehen werden, daß Tests dieser Art auf viele verschiedene Antigene und Antikörper anwendbar sind.

## Ansprüche

1. Verfahren zur Ermittlung eines Antigens oder Antikörpers in einer flüssigen Probe, bei dem man die Probe mit einer porösen Membran, an die ein entsprechender Antikörper bzw. entsprechendes Antigen gebunden ist und an die sich ein Flüssigkeit aufnehmendes poröses Absorbensmaterial anschließt, und einem sich an das Antigen bzw. den Antikörper bindenden konjugierten oder gelabelten. Antikörper in Berührung bringt und gegebenenfalls diese Antikörper unter Bildung eines Farbstoffes entwickelt, dadurch gekennzeichnet, daß man in einem Behälter ein solches Volumen der flüssigen Probe, daß mit ihm das Porenvolumen des Absorbensmaterials nur teilweise füllbar ist, vorlegt, die Membran mit dem daran anschließenden Absorbensmaterial derart in die flüssige Probe eintaucht, daß diese im wesentlichen vollständig oder mit vorbestimmtem Volumen entgegen der Schwerkraft durch die Membran in das Absorbensmaterial gesaugt wird, und gegebenenfalls schließlich nach Entfernung der Membran aus der Probe sie in eine Entwicklerlösung eintaucht und diese entgegen der Schwerkraft in das Absorbensmaterial einsaugen läßt.

2. Verfahren nach Anspruch 1, dadurch gekennzeichnet, daß man die flüssige Probe vor dem Einsaugen durch die Membran filtriert.

3. Verfahren nach einem der Ansprüche 1 und 2, dadurch gekennzeichnet, daß man nach dem Einsaugen der flüssigen Probe und vor dem Einsaugen der Entwicklerlösung die Membran von überschüssigem Probenmaterial und/oder konjugiertem oder gelabeltem Antikörper freispült.

4. Verfahren nach einem der Ansprüche 1 bis 3, dadurch gekennzeichnet, daß man die flüssige Probe mit dem konjugierten oder gelabelten Antikörper darin vorlegt.

5. Verfahren nach einem der Ansprüche 1 bis 3, dadurch gekennzeichnet, daß man die flüssige Probe ohne den konjugierten oder gelabelten Antikörper darin vorlegt und nach deren Einsaugen die Membran in eine Lösung des konjugierten oder gelabelten Antikörpers eintaucht und diese einsaugt.

6. Vorrichtung zur Ermittlung eines Antigens oder Antikörpers in einer flüssigen Probe mit einem porösen Membranteil, an das ein sich an das Antigen bindender Antikörper bzw. an den Antikörper bindendes Antigen gebunden ist, und einer sich an das Membranteil anschließenden Säule eines porösen Absorbensmaterials, gekennzeichnet durch einen Probenbehälter (1, 10) und einen rohrförmigen Absorbensmaterial-Behälter (3), der an einem Ende durch das Membranteil (5) verschlossen und am anderen Ende mit wenigstens einer Öffnung verstehen ist, wobei Probenbehälter (1, 10) und Absorbensmaterial-Behälter (3) so bemessen sind, daß der Absorbenmaterial-Behälter mit der Membran (5) nach unten in den Probenbehälter eintauchbar ist.

7. Vorrichtung nach Anspruch 6, dadurch gekennzeichnet, daß das Absorbensmaterial (7) und die Membran (5) voneinander getrennt sind und zwischen ihnen ein Stützkörper (6) angeordnet ist (Fig. 1).

8. Vorrichtung nach Anspruch 6, dadurch gekennzeichnet, daß das Absorbensmaterial (7) eine Säule bildet und einstückig mit dem Membranteil (5) verbunden ist (Fig. 2).

9. Vorrichtung nach einem der Ansprüche 6 bis 8, dadurch gekennzeichnet, daß der Antikörper bzw. das Antigen nur an einen Teilbereich (12) oder Oberfläche des Membranteils (5) gebunden ist, während an einen anderen Teilbereich (13) des Membranteils (5) das Antigen oder ein anderer Antikörper gebunden ist.

10. Vorrichtung nach einem der Ansprüche 6 bis 9, dadurch gekennzeichnet, daß dem Membranteil in Strömungsrichtung der Probe entgegen der Schwerkraft ein Filter (9) vorgeschaltet ist.

11. Vorrichtung nach Anspruch 10, dadurch gekennzeichnet, daß der Filter (9) an dem Absorbensmaterial-Behälter (3) oder in dem Probenbehälter (10) befestigt ist.

12. Vorrichtung nach Anspruch 6, gekennzeichnet durch einen Außenbehälter (31), einen in den Außenbehälter einsetzbaren Probenbehälter (35), den in den Probenbehälter (35) einsetzbaren und an einem auf dem Außenbehälter (31) aufsetzbaren Halteteil (24) befestigten Absorbensmaterial-Behälter (25) sowie einen in das Halteteil (24) und einen Fortsatz (18) des Probenbehälters (35) eingreifenden, auf den Außenbehälter (31) aufsetzbaren, abnehmbaren Abstandshalter (20), der den Fortsatz (18) des Probenhälters (35) mit dem Halteteil (24) lösbar verbindet, wobei die Abmessungen von Außenbehälter (31), Abstandshalter (20) und Absorbensmaterial-Behälter (25) derart sind, daß das Membranteil bei aufgesetztem Abstandshalter oberhalb des Probenbehälters (35) und bei abgenommenem Abstandshalter in dem Probenbehälter angeordnet ist.

13. Vorrichtung nach Anspruch 12, dadurch gekennzeichnet, daß der Abstandshalter (20) im wesentlichen U-förmig ausgebildet ist und mit einer ersten Nut (21) oder Feder in eine Feder (23) bzw. Nut am Halteteil (24) und mit einer zweiten Nut (12) oder Feder in eine Feder (19) bzw. Nut am oberen Bereich des Fortsatzes (18) des Probenbehälters (35) eingreift.

14. Vorrichtung nach Anspruch 12 oder 13, dadurch gekennzeichnet, daß der obere Rand des im wesentlichen rohrförmigen Fortsatzes (18) des Probenbehälters (35) eine Wulst (19) besitzt, die auf einer Schulter (12) an der Innenseite des Abstandshalters (20) aufliegt.

15. Vorrichtung nach einem der Ansprüche 12 bis 14, dadurch gekennzeichnet, daß der Außenbehälter (31) zusätzlich einen Entwicklerbehälter (33) enthält, in den der Absorbensmaterial-Behälter (25) einfügbar ist.

16. Vorrichtung nach einem der Ansprüche 12 bis 15, dadurch gekennzeichnet, daß der Probenbehälter (35) einen unteren becherförmigen, seitlich mit Flüssigkeitseintrittsöffnungen (36) versehenen Endabschnitt eines rohrförmigen Fortsatzes (18) bildet.

17. Vorrichtung nach Anspruch 16, dadurch gekennzeichnet, daß der rohrförmige Fortsatz (18) zwischen den seitlichen Flüssigkeitseintrittsöffnungen (36) in seiner Längsrichtung verlaufende Ablaufrippen (17) aufweist.

18. Vorrichtung nach einem der Ansprüche 12 bis 17, dadurch gekennzeichnet, daß das Halteteil (24) als Deckel ausgebildet ist.

19. Vorrichtung nach einem der Ansprüche 12 bis 18, dadurch gekennzeichnet, daß das Halteteil (24) und der Abstandshalter (20) auf die Öffnung des Außenbehälters (31) aufgepaßt sind.

Fig. 3

Fig. 2

Fig. 1

Fig. 4

Fig. 5

Fig. 6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 046 004 (SYVA CO.)<br>* Seiten 1-11; Seite 19, Zeilen 22-37; Seite 23, Zeile 22 - Seite 29, Zeile 32 * | 1,3-6,8,10 | G 01 N 33/543<br>B 01 L 3/00 |
| | --- | | |
| A | US-A-4 246 339 (F.X. COLE et al.)<br>* Figuren; Spalte 1, Zeile 1 - Spalte 3, Zeile 43 * | 1,3,5 | |
| A | EP-A-0 191 640 (SYNTEX (U.S.A.) INC.)<br>* Seite 4, Zeile 10 - Seite 7, Zeile 34; Seite 19, Zeile 4 - Seite 23, Zeile 20 * | 1,3-5 | |
| | --- | | |
| A | WO-A-8 505 451 (HYBRITECH INC.)<br>* Figur 1; Seite 3, Zeile 5 - Seite 4, Zeile 31 * & US-A-4 632 901 (Kat. D) | 1-5 | |
| | --- | | |
| A | FR-A-2 379 072 (AMES-YISSUM LTD)<br>* Ansprüche 1-8 * | 1 | |
| | ----- | | |

**RECHERCHIERTE SACHGEBIETE (Int. Cl.4)**

G 01 N
B 01 L

Der vorliegende Recherchenbericht wurde für alle Patentansprüche erstellt

| Recherchenort | Abschlußdatum der Recherche | Prüfer |
|---|---|---|
| DEN HAAG | 16-11-1988 | HITCHEN C.E. |